# EUROPEAN PATENT APPLICATION

(11) **EP 4 537 887 A2**
(43) Date of publication of application: **16.04.2025**
(21) Application number: 24215434.2
(22) Date of filing: 10.06.2021
(51) Int. Cl.: A61M 25/10

(54) **SYSTEM FOR EXPANDING IMPLANTS**

(30) Priority: 11.06.2020 US 202063038035 P
(62) Divisional of application: 21736904.0
(71) Applicant: Edwards Lifesciences Corporation, Irvine, CA 92614 (US)
(72) Inventor: LEVI, Tamir S., Irvine, CA 92614 (US)
(74) Representative: Eisenführ Speiser

(57) **Abstract**

The present embodiments relate generally to devices, systems, and methods for expansion of an expandable implant. A system may include a first inflatable body having a first outer diameter when in an inflated state. The system may include a second inflatable body positioned adjacent to the first inflatable body and having an outer surface configured to apply an expansion force to the expandable implant and having a shape that tapers downward in a direction towards the first inflatable body to a narrow portion having a second outer diameter that is less than the first outer diameter when the second inflatable body is in an inflated state.

## Description

### RELATED APPLICATION

This application claims the benefit of and priority to U.S. Provisional Patent Application Serial No. 63/038,035, filed on June 11, 2020, which is incorporated herein in its entirely by this specific reference.

### BACKGROUND OF THE INVENTION

A variety of maladies may affect an individual's body. Such maladies may be of the individual's heart, and may include maladies of the individual's heart valves, including the aortic, mitral, tricuspid, and pulmonary valves. Stenosis, for example, is a common and serious valve disease that may affect the operation of the heart valves and an individual's overall well-being.

Implants may be provided that may replace or repair portions of a patient's heart. Prosthetic implants, such as prosthetic heart valves, may be provided to replace a portion of a patient's heart. Prosthetic aortic, mitral, tricuspid, and even pulmonary valves may be provided.

Implants may be deployed to the desired portion of the patient's body percutaneously, in a minimally invasive manner. Such deployment may occur transcatheter, in which a catheter may be deployed through the vasculature of an individual.

During deployment of such implants, the implants must be expanded to provide an expanded configuration for such implant. Care must be taken to properly expand the implants to a desired implantation site, and to avoid over expansion or under expansion of such implants.

### SUMMARY

Expandable implants may be expanded by inflatable bodies, which may comprise balloons or another form of inflatable body. Upon expansion of the expandable implants by the inflatable bodies, care must be taken to assure that the expandable implant is positioned in the desired location upon the inflatable body, to provide the desired implantation location and expansion size of the expandable implant. These concerns may be increased with "V" shaped implants, as "V" shaped implants might slip upon the inflatable body and produce an undesired position of the implant upon the inflatable body. Further, "V" shaped implants may have an expansion size that depends on the position of the implant upon an inflatable body.

Embodiments disclosed herein may be directed to improved positioning of an expandable implant upon one or more inflatable bodies upon expansion of the inflatable bodies. Embodiments may be utilized with a "V" shaped implant. Embodiments as disclosed herein may include a system for expansion of an expandable implant. The system may include a first inflatable body having a first outer diameter when in an inflated state. The system may include a second inflatable body positioned adjacent to the first inflatable body and having an outer surface configured to apply an expansion force to the expandable implant and having a shape that tapers downward in a direction towards the first inflatable body to a narrow portion having a second outer diameter that is less than the first outer diameter when the second inflatable body is in an inflated state.

Embodiments as disclosed herein may include a delivery system for an expandable implant. The delivery system may include a delivery apparatus configured to deliver the expandable implant to a location in a patient's body. The delivery apparatus may include an elongate shaft and a first inflatable body coupled to the elongate shaft and having a first outer diameter when in an inflated state. The delivery apparatus may include a second inflatable body coupled to the elongate shaft adjacent to the first inflatable body and having an outer surface configured to apply an expansion force to the expandable implant and having a shape that tapers downward in a direction towards the first inflatable body to a narrow portion having a second outer diameter that is less than the first outer diameter when the second inflatable body is in an inflated state.

Embodiments as disclosed herein may include a method. The method may include inflating a first inflatable body. The method may include radially expanding an expandable implant positioned around an outer surface of a second inflatable body by inflating the second inflatable body, the second inflatable body being positioned adjacent to the first inflatable body and having an outer surface having a shape that tapers downward in a direction towards the first inflatable body to a narrow portion, with an outer diameter of the narrow portion being less than an outer diameter of the first inflatable body.

### BRIEF DESCRIPTION OF THE DRAWINGS

These and other features, aspects, and advantages are described below with reference to the drawings, which are intended to illustrate, but not to limit, the disclosure. In the drawings, like reference characters denote corresponding features consistently throughout similar embodiments.
FIG. 1 is a cross sectional view of a system for expansion of an expandable implant according to an embodiment of the present disclosure.
FIG. 2 is a cross sectional view of the system shown in FIG. 1, with an inflatable body inflated.
FIG. 3 is a cross sectional view of the system shown in FIG. 1, with two inflatable bodies inflated.
FIG. 4 is a cross sectional view of the system shown in FIG. 1, with two inflatable bodies inflated.
FIG. 5 is a perspective view of the system shown in FIG. 4, with the implant removed from view.
FIG. 6 is a side view of an expandable implant according to an embodiment of the present disclosure.
FIG. 7 is a cross sectional view of the expandable implant shown in FIG. 6 according to an embodiment of the present disclosure.
FIG. 8 is a cross sectional view of a system for expansion of an expandable implant according to an embodiment of the present disclosure.
FIG. 9 is a cross sectional view of a system for expansion of an expandable implant according to an embodiment of the present disclosure.
FIG. 10 is a side view of a delivery apparatus according to an embodiment of the present disclosure.
FIG. 11 is a schematic view of a delivery apparatus approaching an aortic valve.
FIG. 12 is a schematic view of a system for expansion of an expandable implant having an inflatable body inflated.
FIG. 13 is a schematic view of the system for expansion of an expandable implant shown in FIG. 12 having two inflatable bodies inflated.
FIG. 14 is a schematic view of a deployed implant according to an embodiment of the present disclosure.

### DETAILED DESCRIPTION

The following description and examples illustrate some example embodiments of the disclosure in detail. Those of skill in the art will recognize that there are numerous variations and modifications of the disclosure that are encompassed by its scope. Accordingly, the description of a certain example embodiment should not be deemed to limit the scope of the present disclosure.

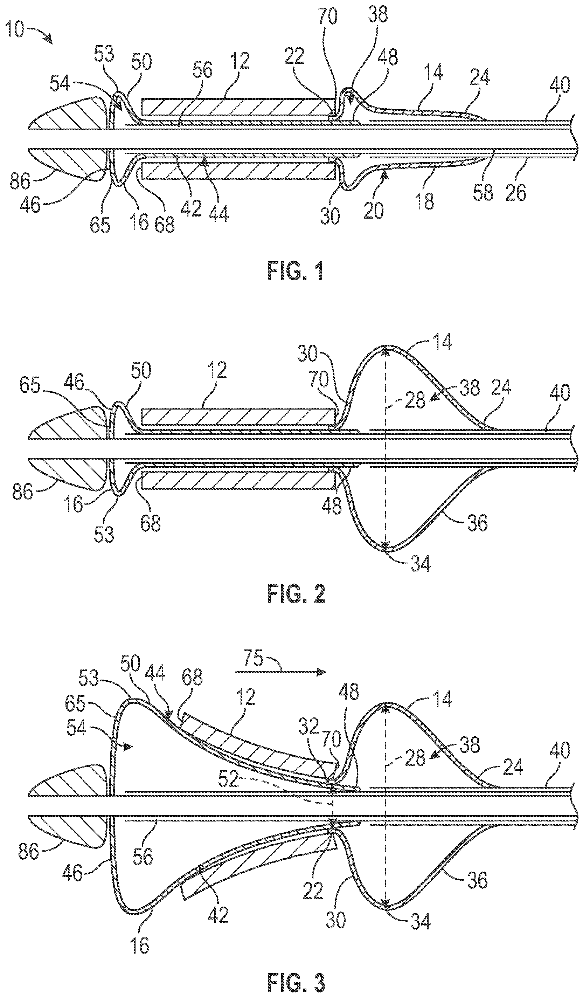

FIG. 1 illustrates a side cross sectional view of a system 10 for expansion of an expandable implant 12. The system may include an inflatable body 14, which may be referred to as a first or proximal inflatable body, and may include an inflatable body 16, which may be referred to as a second or distal inflatable body.

The inflatable body 14 may include an outer wall 18 forming an outer surface 20 of the inflatable body 14. The inflatable body 14 may have a first end 22 and a second end 24. The first end 22 may be coupled to a portion of the second inflatable body 16, and the second end 24 may be coupled to an elongate shaft 26 of a delivery apparatus configured to deliver the expandable implant 12 to a location in a patient's body. The inflatable body 14 may extend axially along the length of the elongate shaft 26 of the delivery apparatus in both a deflated state as shown in FIG. 1 and in an inflated state as shown in FIGS. 2-4.

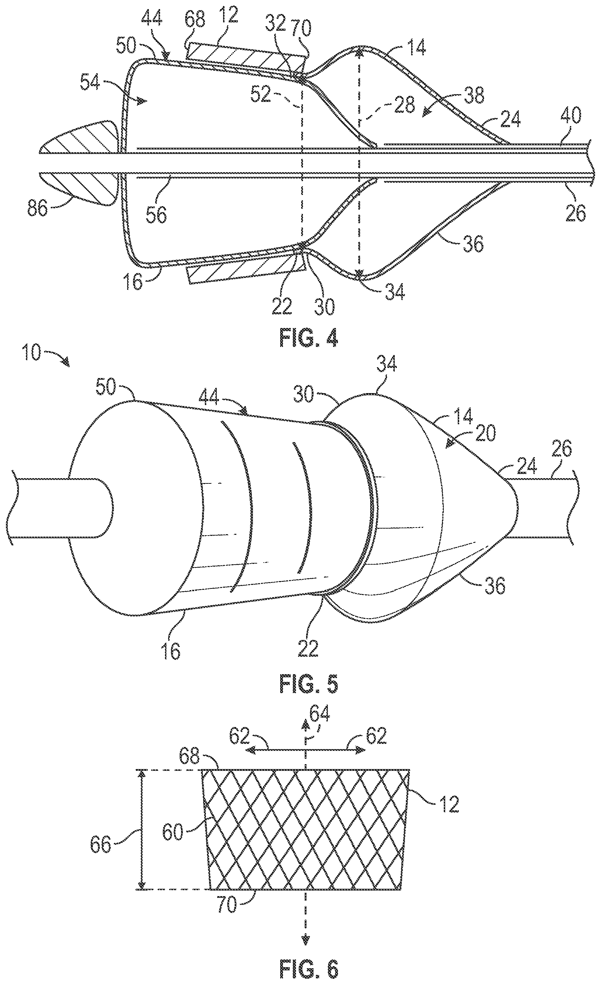

The inflatable body 14 may be configured to have a rounded profile when in an inflated state. Such a profile is shown for example in FIGS. 2-4. The inflatable body 14 may extend radially outward from the elongate shaft 26 when in an inflated state, and may extend around the axis of the elongate shaft 26. The inflatable body 14 may have a bulb shape as shown in FIG. 5 for example when in an inflated state, or may have another shape as desired. The inflatable body 14 may have an outer diameter 28 (as shown in FIGS. 2-4) when in an inflated state.

The inflatable body 14 may be configured to have a shoulder portion 30. The shoulder portion 30 may be positioned proximate to a narrow portion 32 of the second inflatable body 16 (as marked in FIGS. 3 and 4). When the inflatable body 14 is in a deflated state, as shown in FIG. 1, for example, the shoulder portion 30 may comprise a bump formed by the outer wall 18 of the inflatable body 14. The bump may be formed by the outer wall 18 being a folded portion, as shown in FIG. 1, or the outer wall 18 may include a thicker portion at the shoulder portion 30 to form the bump in embodiments as desired. As shown in FIGS. 2-4, as the inflatable body 14 is inflated, the shoulder portion 30 may form an outwardly extending and curved surface extending to an apex 34 of the inflatable body 14. The inflatable body 14 may include a downwardly tapering portion 36 on the opposite side of the apex 34, extending downward to the second end 24 of the inflatable body 14.

The outer wall 18 of the inflatable body 14 may surround an interior chamber 38 of the inflatable body 14 that may be configured to hold fluid (for example, a liquid or other fluid in embodiments) for inflating the inflatable body 14. The interior chamber 38 may comprise a single chamber as shown in FIGS. 1-4, or a plurality of chambers utilized to hold fluid for inflating the inflatable body 14. The interior chamber 38 may be sealed from outside of the inflatable body 14 by the coupling of the ends 22, 24 of the inflatable body 14 to the inflatable body 16 and the elongate shaft 26, respectively.

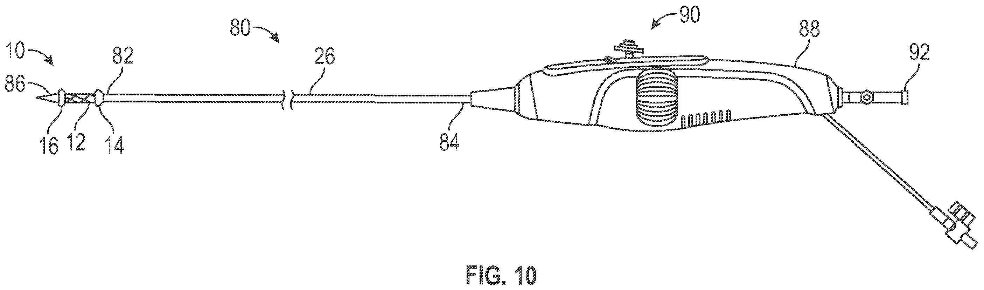

An inflation lumen 40 may be provided for passing fluid into the interior chamber 38 of the inflatable body 14. The inflation lumen 40 may extend along the elongate shaft 26 and may have a proximal end that couples to a port 92 (as shown in FIG. 10) for passing fluid into and out of the inflation lumen 40, and may have a distal end with an opening for passing fluid into and out of the interior chamber 38 of the inflatable body 14. The configuration of the inflation lumen 40 may be varied in other embodiments.

The inflatable body 16 (or second or distal inflatable body), similar to the inflatable body 14, may include an outer wall 42 forming an outer surface 44 of the inflatable body 16. The outer surface 44 of the inflatable body 16 may be configured to apply an expansion force to the expandable implant 12. The inflatable body 16 may have a first end 46 and a second end 48. The first end 46 and second end 48 may each be coupled to the elongate shaft 26 of the delivery apparatus for the expandable implant 12. The inflatable body 16 may extend axially along the length of the elongate shaft 26 of the delivery apparatus in both a deflated state as shown in FIGS. 1-2 and in an inflated state as shown in FIGS. 3-4.

The inflatable body 16 may extend radially outward from the elongate shaft 26 when in an inflated state, and may extend around the axis of the elongate shaft 26. The inflatable body 16 may have a conical frustum shape as shown in FIG. 5 for example when in an inflated state, or may have another shape as desired.

The inflatable body 16 may include a shoulder portion 50 that may be positioned proximate the first end 46 of the inflatable body 16. When the inflatable body 16 is in a deflated state, as shown in FIG. 1, for example, the shoulder portion 50 may comprise a bump formed by the outer wall 42 of the inflatable body 16. The bump may be formed by the outer wall 42 being a folded portion, as shown in FIG. 1, or the outer wall 42 may include a thicker portion at the shoulder portion 50 to form the bump in embodiments as desired. As shown in FIGS. 2-4, as the inflatable body 16 is inflated, the shoulder portion 50 may form an outwardly extending and curved surface that is positioned offset from the expandable implant 12. The shoulder portion 50 may include a surface extending from an apex 53 of the inflatable body 16 in a direction towards the second end 48 of the inflatable body 16. The inflatable body 16 may include a downwardly tapering portion 65 on the opposite side of the apex 53, extending downward to the first end 46 of the inflatable body 16.

The outer surface 44 of the inflatable body may have a shape that tapers downward in a direction towards the inflatable body 14 to a narrow portion 32 (as marked in FIGS. 3 and 4) when the inflatable body 16 is in an inflated state. The narrow portion 32 may have an outer diameter 52 (as shown in FIGS. 3 and 4) when in an inflated state. The outer diameter 52 of the narrow portion 32 may be less than the outer diameter 28 of the inflatable body 14.

The outer wall 42 of the inflatable body 16 may surround an interior chamber 54 of the inflatable body 16 that may be configured to hold fluid (for example, a liquid or other fluid in embodiments) for inflating the inflatable body 16. The interior chamber 54 may comprise a single chamber as shown in FIGS. 1-4, or a plurality of chambers utilized to hold fluid for inflating the inflatable body 16. The interior chamber 54 may be sealed from outside of the inflatable body 16 and from the interior chamber 38 of the inflatable body 14 by the coupling of the ends 46, 48 of the inflatable body 16 to the shaft 26.

An inflation lumen 56 may be provided for passing fluid into the interior chamber 54 of the inflatable body 16. The inflation lumen 56 may extend along the elongate shaft 26 and may have a proximal end that couples to a port 92 (as shown in FIG. 10) for passing fluid into and out of the inflation lumen, and may have a distal end with an opening for passing fluid into and out of the interior chamber 54 of the inflatable body 16. The inflation lumen 56 may be configured to extend around an interior shaft 58 (e.g., a guide wire lumen) of the elongate shaft 26, with the inflation lumen 40 extending around and concentric with the inflation lumen 56. The configuration of the inflation lumen 56 may be varied in other embodiments.

Each inflatable body 14, 16 may be configured to be inflated by fluid passing into the respective chambers 38, 54 and may be configured to be deflated by fluid passing out of the respective chambers 38, 54. The inflatable bodies 14, 16 may be configured to be separately inflated and deflated as desired. The inflatable bodies 14, 16 in embodiments may comprise balloons, which may be non-compliant in embodiments. As such, the inflatable bodies 14, 16 may be pre-formed to have the shapes in an inflated state as shown in FIGS. 4 and 5 for example, and may then be inflated to that shape. In embodiments, the inflatable bodies 14, 16 may be semi-compliant or compliant as desired. The inflatable bodies 14, 16 may be pre-formed with the shapes shown in the inflated state as shown in FIGS. 4 and 5 or may otherwise be configured to form the shapes shown in FIGS. 4 and 5.

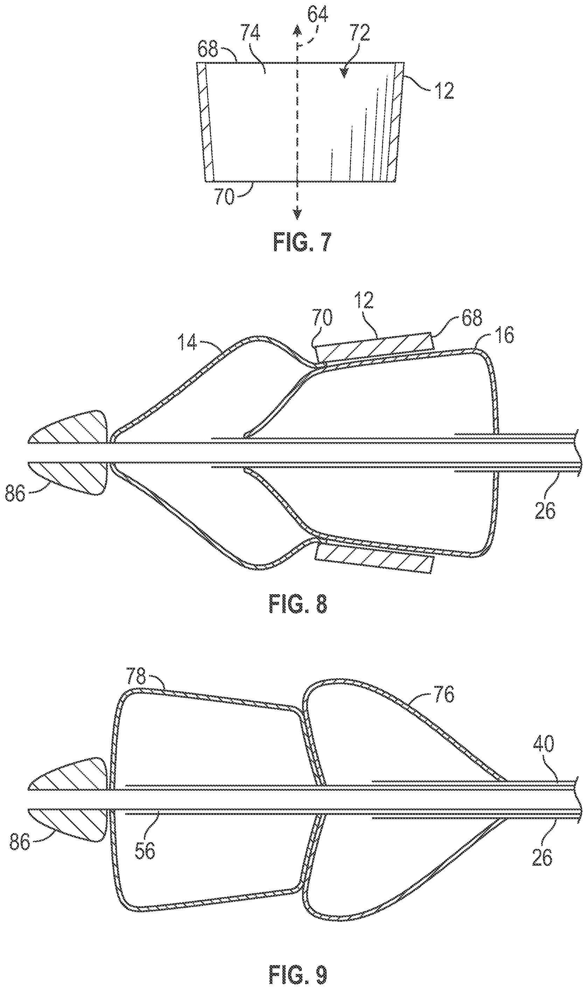

The inflatable bodies 14, 16 may be positioned adjacent to each other. The inflatable bodies 14, 16 may be positioned adjacent to each other axially along the length of the elongate shaft 26. The inflatable bodies 14, 16 may be positioned with the first inflatable body 14 positioned proximal along the length of the elongate shaft 26 and the second inflatable body 16 positioned distal along the length of the elongate shaft 26 as shown in FIGS. 1-5 for example. In embodiments, the inflatable bodies 14, 16 may be positioned with the first inflatable body 14 positioned distal along the length of the elongate shaft 26 and the second inflatable body 16 positioned proximal along the length of the elongate shaft 26 as shown in FIG. 8 for example. The inflatable bodies 14, 16 may positioned adjacent to each other and may be in contact with each other, or a gap may be positioned between the inflatable bodies 14, 16, or another device may be positioned between the inflatable bodies 14, 16.

In an embodiment as shown in FIGS. 1-5, a portion of one of the inflatable bodies 14, 16 may overlap a portion of another of the inflatable bodies 14, 16. For example, as shown in FIG. 1, the first end 22 of the first inflatable body 14 may comprise an overlap portion that overlaps the second inflatable body 16. The first end 22 overlaps and couples to the narrow portion 32 of the second inflatable body 16. The shoulder portion 30 of the first inflatable body 14 further comprises an overlap portion that overlaps the second end 48 of the second inflatable body 16. The overlap of the first inflatable body 14 over the second inflatable body 16 may allow the first inflatable body 14 to increase in size along with the inflation of the second inflatable body 16. For example, as shown in FIGS. 3 and 4, the first end 22 of the first inflatable body 14 is coupled to the narrow portion 32 of the second inflatable body 16 such that an inflation of the narrow portion 32 causes the first end 22 of the first inflatable body 14 to increase in size. In other embodiments, for example as shown in FIG. 9, the inflatable bodies may not be coupled to each other.

The expandable implant 12 may comprise an implant 12 configured to be radially expanded outward via an expansion force applied by the inflatable body 16. FIG. 6, for example, illustrates a side view of such an implant 12, including a frame 60 that is configured to allow the expandable implant 12 to expand. The frame 60 may be configured as a plurality of struts coupled to each other with spaces between the struts. The frame 60 may be configured such that as the implant 12 is expanded radially outward (for example in a direction shown by the arrows 62 extending outward from the implant axis 64) the length 66 of the implant 12 may shorten. In addition, the frame 60 may be configured such that as the implant 12 is radially compressed, the length 66 of the implant 12 may increase.

Notably, the implant 12 may be configured to shorten in a direction. For example, the implant 12 as shown in FIG. 6 may include a first end 68 and a second end 70. Upon the second end 70, for example, being maintained in a position and the first end 68 being free to move, an expansion force against the implant 12 will cause the length 66 of the implant 12 to shorten in a direction towards the second end 70 (with the first end 68 moving towards the second end 70). Similarly, if the first end 68 were maintained in a position and the second end 70 were free to move, an expansion force against the implant 12 will cause the length 66 of the implant 12 to shorten in a direction towards the first end 68 (with the second end 70 moving towards the first end 68).

The expandable implant 12 may have a variety of forms. For example, the expandable implant 12 may be utilized as a prosthetic heart valve, which may be utilized for implantation in the native aortic, mitral, tricuspid, or pulmonary valves. Other forms of expandable implants may be utilized, including stents or other implants.

The expandable implant 12 may be configured to have a tapered profile. FIG. 7, for example, illustrates a cross sectional view of the implant 12. Features of the implant are not shown for clarity. For example, prosthetic heart valve leaflets may not be shown in FIG. 7 for clarity in an embodiment in which the implant 12 is a prosthetic heart valve. The implant 12 has an angled interior profile that faces an interior cavity 72 of the implant 12. The second end 70 is narrower than the first end 68 and the implant 12 has an inner surface 74 forming an angled interior profile from the first end 68 to the second end 70. The implant 12 may be considered a "V" shaped implant or otherwise an implant having a conical frustum interior shape, or may comprise another form of frustum in other embodiments (e.g., pyramidal or another shape). The implant may have an outer profile that is the same as the interior profile, or may be different in other embodiments.

An implant 12 comprising a "V" shaped implant may have a variety of benefits, including having the prosthetic heart valve leaflets open in a direction towards the wide end (e.g., first end 68) of the implant 12. The prosthetic heart valve leaflets accordingly may have a reduced possibility of contacting the inner surface 74 of the implant 12 upon the leaflets moving to the open state. Other benefits may be provided as desired.

In embodiments, implants other than "V" shaped implants may be utilized. For example, a cylindrical shaped implant or other shape of implant may be utilized, which may be expanded to a tapered profile via use of the system 10 shown in FIG. 1.

An issue that may arise upon expanding expandable implants, particularly implants that are expandable with an inflatable body, is producing desired positioning of the expandable implants upon the inflatable body. This issue may produce difficulties in positioning the implant relative to the desired implantation site, and may produce difficulties in producing a desired size of expansion of the expandable implant. This issue may be enhanced with "V" shaped implants, as the "V" shaped implants may slip upon the inflatable body and produce an undesired position of the implant upon the inflatable body. Further, "V" shaped implants may have an expansion size that depends on the position of the implant upon the inflatable body. The system 10 as disclosed herein may reduce the possibility of such issues arising, and may improve the positioning of the expandable implants upon one or more inflatable bodies.

Referring to FIG. 1, the inflatable bodies 14, 16 are shown in a deflated state. The expandable implant 12 is shown crimped onto the outer surface 44 of the inflatable body 16 and positioned around the outer surface 44. The expandable implant 12 may extend longitudinally along the axis of the elongate shaft 26 and over the inflatable body 16. The expandable implant 12 may be crimped upon the inflatable body 16 with a cylindrical outer shape, and with the first end 68 (the wide end as shown in FIG. 6) of the implant 12 positioned proximate the shoulder portion 50 of the second inflatable body 16 and the second end 70 (the narrow end as shown in FIG. 6) of the implant 12 positioned proximate the shoulder portion 30 of the first inflatable body 14. The expandable implant 12 may be in a compressed state and thus may have a length that is greater than a length of the expandable implant 12 in an expanded state.

The first inflatable body 14 may be positioned axially offset from the expandable implant 12. The first end 22 of the first inflatable body 14 may however be sandwiched between the second end 70 of the expandable implant 12 and the second inflatable body 16 as shown in FIG. 1. The shoulder portion 50 of the second inflatable body 16 may be axially offset from the expandable implant 12. As such, the implant 12 may not cover a portion of the first inflatable body 14 or the second inflatable body 16 in embodiments, with a portion of the first inflatable body 14 or the second inflatable body 16 being axially offset from the implant 12.

With the inflatable bodies 14, 16 in the deflated state as shown in FIG. 1, the shoulder portion 30 of the first inflatable body 14 is positioned adjacent to the second end 70 of the expandable implant 12. The shoulder portion 30 may comprise a protrusion extending radially outward from the elongate shaft 26 of the delivery apparatus. The shoulder portion 30 may accordingly maintain the position of the second end 70 of the expandable implant 12, with the second end 70 of the expandable implant 12 impeded from sliding in a direction towards the inflatable body 14. The shoulder portion 30 may contact the second end 70 of the expandable implant 12 to impede movement in the direction towards the inflatable body 14. The shoulder portion 30 may have a diameter that is greater than a diameter of the implant 12 as shown in FIG. 1.

Further, as shown in FIG. 1, the shoulder portion 50 of the second inflatable body 16 may be positioned adjacent to the first end 68 of the expandable implant 12. The shoulder portion 50 may comprise a protrusion extending radially outward from the elongate shaft 26 of the delivery apparatus. The shoulder portion 50 may accordingly impede the first end 68 of the expandable implant 12 from sliding in a direction away from the first inflatable body 14. The shoulder portion 50 may contact the first end 68 of the expandable implant 12 to impede movement in the direction away from the first inflatable body 14. The shoulder portion 50 may have a diameter that is greater than a diameter of the implant 12 as shown in FIG. 1.

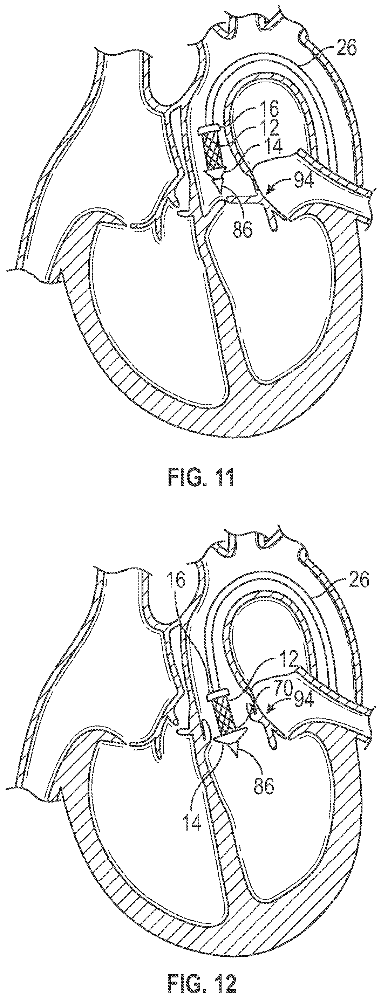

In operation, the expandable implant 12 may be delivered to a desired location within a patient's body such as an implantation site while being positioned upon the second inflatable body 16, and with the system 10 in the configuration shown in FIG. 1. The elongate shaft 26 may be moved to the desired implantation site as shown, for example in FIGS. 11-13, or via another method as desired.

Upon the expandable implant 12 being delivered to a desired implantation site, or prior to such movement, the first inflatable body 14 may be inflated as shown in FIG. 2. Referring to FIG. 2, fluid may be passed through the inflation lumen 40 and into the interior chamber 38 of the first inflatable body 14. The first inflatable body 14 may be in an inflated state, and may have an increased outer diameter 28 of the inflatable body 14. The size of the shoulder portion 30 may further increase. Notably, the shoulder portion 30 may continue to maintain the position of the second end 70 of the expandable implant 12, by continuing to impede movement of the second end 70 of the expandable implant 12 in a direction towards the first inflatable body 14. The second end 70 of the expandable implant 12 may remain in contact with the surface of the shoulder portion 30.

Upon the first inflatable body 14 being inflated, the second inflatable body 16 may be at least partially inflated. FIG. 3, for example, illustrates the second inflatable body 16 in an inflated state, being partially inflated. Fluid may be passed through the inflation lumen 56 and into the interior chamber 54 of the second inflatable body 16 to inflate the second inflatable body 16. Upon inflation, the shoulder portion 50 of the second inflatable body 16 may be configured to first inflate, with inflation continuing in a direction towards the first inflatable body 14. The increased diameter of the shoulder portion 50 may impede movement of the first end 68 of the expandable implant 12 in a direction away from the first inflatable body 14. Further, the tapered shape of the outer surface 44 of the second inflatable body 16 may cause the outer surface 44 to apply an expansion force against the expandable implant 12 causing the first end 68 of the expandable implant 12 to move towards the first inflatable body 14.

As discussed in regard to FIG. 2, the shoulder portion 30 may continue to maintain the position of the second end 70 of the expandable implant 12, by continuing to impede movement of the second end 70 of the expandable implant 12 in a direction (as indicated with arrow 75 in FIG. 3) towards the first inflatable body 14. As such, the outer surface 44 of the first inflatable body 14 may continue to apply a force to the expandable implant 12, with the shoulder portion 30 providing a resistive counter force that maintains the position of the second end 70 of the expandable implant 12. The first inflatable body 14 accordingly may comprise a stopper to impede movement of the second end 70 of the expandable implant 12. As discussed in regard to FIG. 6, with the first end 68 of the expandable implant 12 being free to move towards the first inflatable body 14 and the second end 70 being maintained in position, the expandable implant 12 shortens in a direction towards the second end 70 upon being radially expanded. Such movement is further enhanced by the direction of taper of the outer surface 44. The expandable implant 12 may be configured to expand radially outward and have the length 66 of the expandable implant 12 shorten in a direction towards the first inflatable body 14 when the outer surface 44 applies the expansion force to the expandable implant 12.

The tapered shape of the outer surface 44 includes a narrow portion 32 having a diameter 52. The diameter 28 of the first inflatable body 14 is greater than the diameter 52 of the narrow portion 32, thus allowing the first inflatable body 14 to maintain the position of the second end 70 of the expandable implant 12.

The second inflatable body 16 may continue to be inflated, with the expandable implant 12 radially expanded and a length of the expandable implant 12 continuing to shorten in a direction towards the second end 70 of the implant 12. The expandable implant 12 may be radially expanded by inflating the second inflatable body 16. FIG. 4, for example, illustrates the second inflatable body 16 in a fully inflated state, with the expandable implant 12 shortened in a direction towards the first inflatable body 14. The outer surface 44 continues to apply an expansion force upon the implant 12. The shoulder portion 30 of the first inflatable body 14 continues to maintain a position of the second end 70 of the implant during radial expansion of the implant 12 by impeding movement of the second end 70 of the expandable implant 12 in a direction towards the first inflatable body 14. The narrow portion 32 of the second inflatable body 16 has increased in size, yet remains smaller in diameter 52 than the diameter 28 of the first inflatable body 14. The implant 12 may be fully deployed, and may have a tapered shape when radially expanded.

The axial position of the implant 12 upon the tapered outer surface 44 of the second inflatable body 16 defines the expansion diameter of the implant 12. As such, with a defined position of the implant 12 upon the tapered outer surface 44, the expansion diameter of the implant 12 may be known. The shape of outer surface 44 of the second inflatable body 16 may be defined to produce a desired tapered shape of the implant 12 upon expansion, as well as the expansion diameter of the implant 12.

Upon the implant 12 being fully deployed, the inflatable bodies 14, 16 may be deflated in a reverse sequence than shown in FIGS. 2-4. The inflatable bodies 14, 16 and the elongate shaft 26 may be removed from the implantation site, with the deployed implant 12 remaining in position.

The system 10 as disclosed herein may provide a variety of benefits, including improved positioning of the implant 12 upon the inflatable body 16 and deployment of the implant 12 from the inflatable body 16. The first inflatable body 14 may serve to impede movement of the second end 70 of the expandable implant 12 towards the first inflatable body 14, thus defining a position of the second end 70 of the expandable implant upon deployment. As such, the second end 70 of the implant 12 may be aligned with the desired implantation site and will be impeded from moving undesirably proximally from this position. The implant 12 may be deployed with the position of the second end 70 of the implant being defined, thus reducing the possibility of undesired positioning of the implant 12 upon the inflatable body 16. Further, undesired slippage or other undesired processes in the deployment process may be reduced.

FIG. 5 illustrates a perspective view of the system 10 in the configuration shown in FIG. 4, with the expandable implant 12 excluded from view for clarity. The first inflatable body 14 is shown to have a bulb shape, and the second inflatable body 16 is shown to have a conical frustum shape.

Variations in the configuration of the system 10 may be provided as desired. FIG. 8, for example, illustrates a side cross sectional view of an embodiment of the system in which the positions of the first inflatable body 14 and the second inflatable body 16 are reversed from the positions shown in FIG. 1 upon the elongate shaft 26 of the delivery apparatus. The second inflatable body 16 may be positioned proximal and the first inflatable body 14 may be positioned distal. Such a reversed configuration may allow for a different delivery orientation of the expandable implant 12 to the desired implantation site. For example, the wide end 68 of the expandable implant 12 may be positioned proximal and the narrow end 70 may be positioned distal. Such a reversed configuration may also allow for a different direction of delivery approach to the implantation site. For example, with regard to an aortic heart valve, one configuration may allow for a transvascular approach (e.g., over the aortic arch) and another reversed configuration may allow for a transapical approach. Various other approaches may be utilized for an implantation site as desired.

Other variations may be utilized. For example, FIG. 9 illustrates an embodiment in which an inflatable body 76 is not coupled to an adjacent inflatable body 78. The inflatable body 76 may otherwise be configured similarly as the first inflatable body 14 shown in FIG. 1, and the inflatable body 78 may otherwise be configured similarly as the second inflatable body 16 shown in FIG. 1.

Other variations may include a configuration in which a single inflation lumen is utilized to inflate both the first inflatable body and the second inflatable body. The inflation lumen, for example, may include a valve or other device that may allow for selective inflation of the inflatable bodies. One or more inflation lumens may extend along the elongate shaft 26 and may be configured to inflate one or more of the first inflatable body or second inflatable body.

Other variations may include a configuration in which the first inflatable body and the second inflatable body are comprised of a unitary body. The first inflatable body, for example, may be made of a material that more easily inflates than the second inflatable body. Upon inflation, the first inflatable body may then inflate first, due to the relatively reduced force at which the first inflatable body inflates. As the first inflatable body reaches its maximum size, the resistance to inflation of the first inflatable body may increase, and thus the second inflatable body may begin to expand due to inflation. The second inflatable body may then inflate until it reaches its maximum size. In this manner, the first inflatable body and second inflatable body may utilize a single fluid chamber and a single inflation lumen may be utilized. The sequence of the first inflatable body being inflated first and the second inflatable body being inflated second may be maintained due to the different materials of the first inflatable body and the second inflatable body, or other configuration of the bodies.

In embodiments, portions of the first inflatable body and second inflatable body may be covered with materials. For example, coatings or other coverings may be positioned over the inflatable bodies. A coating may cover the outer surface of the second inflatable body, yet the outer surface may apply an expansion force to the expandable implant through the coating. Combinations of features across various embodiments and other variations may be utilized as desired.

The system may be utilized as part of a delivery system for the expandable implant. FIG. 10, for example, illustrates a delivery apparatus 80 that may be utilized to deliver the expandable implant 12 to a location in a patient's body. The delivery apparatus 80 may include the elongate shaft 26, which may have a distal portion 82 and a proximal portion 84. The system 10 including the inflatable bodies 14, 16 may be positioned on the distal portion 82 of the elongate shaft 26. The elongate shaft 26 may include a nose cone 86, which may couple to an interior shaft 58 of the elongate shaft 26 (as shown in FIGS. 1-4). The interior shaft 58 may comprise a guide wire lumen for a guide wire to extend through as the delivery apparatus 80 approaches an implantation site. The nose cone 86 may be positioned distal of the inflatable bodies 14, 16. In embodiments, the second inflatable body 16 may be positioned adjacent to the nose cone 86 (or the first inflatable body 14 may be positioned adjacent to the nose cone 86 in an embodiment as shown in FIG. 8).

The proximal portion 84 of the elongate shaft 26 may be coupled to a housing in the form of a handle 88. The handle 88 may be configured to be gripped by a user to control movement of the elongate shaft 26. The delivery apparatus 80 may include an actuation mechanism 90 for actuating operation of the delivery apparatus 80, which may include deflecting the elongate shaft 26 into a desired orientation. For example, the elongate shaft 26 may be configured to be flexible to deflect to the desired portion of the patient's body, and may be steerable with operation of the actuation mechanism 90.

A proximal end of the delivery apparatus 80 may include a port 92 for passing fluid into and out of one or more of the inflation lumens 40, 56.

The configuration of the delivery apparatus may vary from the configuration shown in FIG. 10. Other types of delivery apparatuses may be utilized than shown in FIG. 10.

FIGS. 11-14 illustrate an exemplary method of utilizing systems disclosed herein. Methods disclosed herein may vary from the steps shown in FIGS. 11-14. Referring to FIG. 11, the systems may be utilized in the deployment of an expandable implant 12 that is a prosthetic heart valve. The prosthetic heart valve may comprise a prosthetic aortic heart valve, or in other embodiments may comprise another form of prosthetic heart valve such as a mitral, tricuspid, or pulmonary heart valve. The implant in other embodiments may be utilized for repair, which may comprise repair of a portion of a heart, including heart valve repair. The implant may comprise a "V" shaped implant as shown in FIGS. 6 and 7. The implant in other embodiments may comprise other forms of medical implants including stents, among others.

FIG. 11 illustrates a step in a method of replacing an aortic heart valve. A delivery apparatus 80 for example as shown in FIG. 10 may be utilized to approach the native aortic heart valve 94. The elongate shaft 26 may be deflected to allow the "V" shaped implant 12 to approach the native aortic heart valve 94 through the aortic arch.

The inflatable bodies 14, 16 may be in an orientation relative to each other as shown in FIG. 8, with the first inflatable body 14 being positioned distal and the second inflatable body 16 being positioned proximal. Such an orientation may allow for an expansion of the implant 12 with the wide end of the "V" shaped implant positioned proximal and the narrow end of the implant positioned distal. The inflatable bodies 14, 16 may be in a deflated state, for example as shown in FIG. 1.

Referring to FIG. 12, the system may be advanced to position the expandable implant 12 in the desired implantation location. The first inflatable body 14 may first be inflated to a diameter as described in regard to FIG. 2. The first inflatable body 14 may include the shoulder portion 30 that may impede movement of the narrow end 70 of the implant 12 in the distal direction. As such, the narrow end 70 of the implant 12 may be aligned and positioned with a desired implantation location for the implant 12.

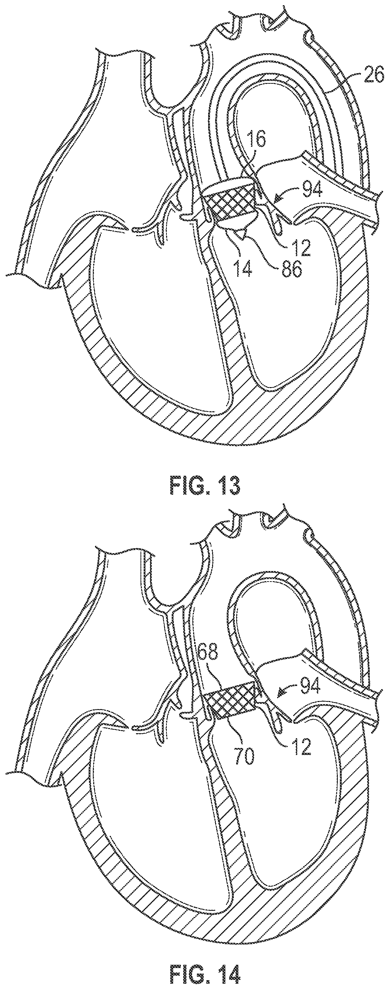

Referring to FIG. 13, upon the first inflatable body 14 being inflated and in the desired position, the second inflatable body 16 may then be subsequently inflated in a process as described in FIGS. 3 and 4. The implant 12 may be expanded and deployed to the implantation site, which is the native aortic valve 94 as shown in FIG. 13. The implant 12 may be positioned between the leaflets of the native aortic valve 94. The wide end 68 of the implant 12 may be positioned proximal and the narrow end 70 of the implant may be positioned distal.

The inflatable bodies 14, 16 may then be deflated and removed from the patient's body. The expandable implant 12 may remain deployed within the patient's body at the implantation site as shown in FIG. 14.

The steps of the method disclosed herein may be varied as desired. The steps may be utilized with other embodiments of systems disclosed herein. The delivery apparatus shown in FIG. 10 is disclosed as being utilized, however, other forms of delivery apparatuses may be utilized. The delivery apparatuses may be configured to deploy implants in the form of prosthetic heart valves, or may be configured to deploy the other forms of implants such as stents or filters, or diagnostic devices, among others.

The other forms of implants such as stents or filters, among others, may be configured similarly as the implants disclosed herein. For example, the implants utilized according to embodiments herein may have an angled interior profile as discussed herein, or may have other profiles as desired. The implants may be cylindrical and may have a uniform interior profile in embodiments, for example. The implants may be configured to expand radially outward from an axis that the implant surrounds, for example a longitudinal axis of the implant.

The delivery apparatus and apparatuses and the systems disclosed herein may be used in a variety of procedures, which may include transcatheter aortic valve implantation (TAVI). The delivery apparatus and the systems disclosed herein may be utilized for transarterial access, including transfemoral access, to a patient's heart. In embodiments, the delivery apparatus may be utilized for mitral, tricuspid, and pulmonary replacement and repair as well. The delivery systems may be utilized in transcatheter percutaneous procedures, including transarterial procedures, which may be transfemoral or transjugular. Transapical procedures, among others, may also be utilized.

Methods as disclosed herein may be utilized in locations that do not utilize native valves, including a pulmonary artery and in the vena cava, among other locations (other arteries, blood vessels, or other vasculature of a patient's body, among other portions of a patient's body). An implant such as a stent or other form of implant may be delivered to such portions of the patient's body.

The user as disclosed herein may comprise a surgeon, physician, or other medical professional, among other users.

Features of embodiments may be modified, substituted, excluded, or combined.

In addition, the methods herein are not limited to the methods specifically described, and may include methods of utilizing the systems and apparatuses disclosed herein.

The steps of the method may be modified, excluded, or added to, with systems, apparatuses, and methods disclosed herein.

The features of the embodiments disclosed herein may be implemented independently of the delivery apparatuses, or independent of other components disclosed herein. The various apparatuses of the systems may be implemented independently.

In closing, it is to be understood that although aspects of the present specification are highlighted by referring to specific embodiments, one skilled in the art will readily appreciate that these disclosed embodiments are only illustrative of the principles of the subject matter disclosed herein. Therefore, it should be understood that the disclosed subject matter is in no way limited to a particular methodology, protocol, and/or reagent, etc., described herein. As such, various modifications or changes to or alternative configurations of the disclosed subject matter can be made in accordance with the teachings herein without departing from the spirit of the present specification. Lastly, the terminology used herein is for the purpose of describing particular embodiments only, and is not intended to limit the scope of systems, apparatuses, and methods as disclosed herein, which is defined solely by the claims. Accordingly, the systems, apparatuses, and methods are not limited to that precisely as shown and described.

Certain embodiments of systems, apparatuses, and methods are described herein, including the best mode known to the inventors for carrying out the same. Of course, variations on these described embodiments will become apparent to those of ordinary skill in the art upon reading the foregoing description. The inventor expects skilled artisans to employ such variations as appropriate, and the inventors intend for the systems, apparatuses, and methods to be practiced otherwise than specifically described herein. Accordingly, the systems, apparatuses, and methods include all modifications and equivalents of the subject matter recited in the claims appended hereto as permitted by applicable law. Moreover, any combination of the above-described embodiments in all possible variations thereof is encompassed by the systems, apparatuses, and methods unless otherwise indicated herein or otherwise clearly contradicted by context.

Groupings of alternative embodiments, elements, or steps of the systems, apparatuses, and methods are not to be construed as limitations. Each group member may be referred to and claimed individually or in any combination with other group members disclosed herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses an approximation that may vary, yet is capable of performing the desired operation or process discussed herein.

The terms "a," "an," "the" and similar referents used in the context of describing the systems, apparatuses, and methods (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the systems, apparatuses, and methods and does not pose a limitation on the scope of the systems, apparatuses, and methods otherwise claimed. No language in the present specification should be construed as indicating any non-claimed element essential to the practice of the systems, apparatuses, and methods.

All patents, patent publications, and other publications referenced and identified in the present specification are individually and expressly incorporated herein by reference in their entirety for the purpose of describing and disclosing, for example, the compositions and methodologies described in such publications that might be used in connection with the systems, apparatuses, and methods. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

The application further comprises the following embodiments:
1. A system for expansion of an expandable implant, the system comprising:
   a first inflatable body having a first outer diameter when in an inflated state; and
   a second inflatable body positioned adjacent to the first inflatable body and having an outer surface configured to apply an expansion force to the expandable implant and having a shape that tapers downward in a direction towards the first inflatable body to a narrow portion having a second outer diameter that is less than the first outer diameter when the second inflatable body is in an inflated state.
2. The system of embodiment 1, wherein the first inflatable body has a rounded profile when in the inflated state.
3. The system of embodiment 1 or embodiment 2, wherein the first inflatable body includes a shoulder portion configured to impede movement of the expandable implant in a direction towards the first inflatable body.
4. The system of embodiment 3, wherein the shoulder portion of the first inflatable body comprises a folded portion of the first inflatable body.
5. The system of any of embodiments 1-4, wherein the first inflatable body has a bulb shape when in the inflated state.
6. The system of any of embodiments 1-5, wherein the second inflatable body includes a shoulder portion configured to impede movement of the expandable implant in a direction away from the first inflatable body.
7. The system of embodiment 6, wherein the shoulder portion of the second inflatable body comprises a folded portion of the second inflatable body.
8. The system of any of embodiments 1-7, wherein the first inflatable body has a first interior chamber, and the system further comprises a first inflation lumen for passing fluid into the first interior chamber.
9. The system of embodiment 8, wherein the second inflatable body has a second interior chamber, and the system further comprises a second inflation lumen for passing fluid into the second interior chamber.
10. The system of embodiment 9, wherein the second interior chamber is sealed from the first interior chamber.
11. The system of any of embodiments 1-10, wherein a portion of the first inflatable body overlaps the second inflatable body.
12. The system of any of embodiments 1-11, wherein the first inflatable body includes a first end and a second end, with the first end coupled to the second inflatable body.
13. The system of embodiment 12, wherein the first end of the first inflatable body is coupled to the narrow portion of the second inflatable body.
14. The system of embodiment 13, wherein the first end of the first inflatable body is coupled to the narrow portion of the second inflatable body such that an inflation of the narrow portion causes the first end of the first inflatable body to increase in size.
15. The system of any of embodiments 12-14, wherein the second end of the first inflatable body is coupled to an elongate shaft of a delivery apparatus configured to deliver the expandable implant to a location in a patient's body.
16. The system of embodiment 15, wherein the second inflatable body includes a first end and a second end each coupled to the elongate shaft of the delivery apparatus.
17. The system of embodiment 15 or embodiment 16, wherein the first inflatable body is positioned on the elongate shaft of the delivery apparatus proximal of the second inflatable body.
18. The system of embodiment 15 or embodiment 16, wherein the first inflatable body is positioned on the elongate shaft of the delivery apparatus distal of the second inflatable body.
19. The system of any of embodiments 15-18, wherein the elongate shaft includes a distal portion and a proximal portion, and the second inflatable body and the first inflatable body are each positioned on the distal portion of the elongate shaft.
20. The system of embodiment 19, wherein the proximal portion of the elongate shaft is coupled to a handle.
21. A delivery system for an expandable implant, the delivery system comprising:
   a delivery apparatus configured to deliver the expandable implant to a location in a patient's body and including:
   an elongate shaft,
   a first inflatable body coupled to the elongate shaft and having a first outer diameter when in an inflated state, and
   a second inflatable body coupled to the elongate shaft adjacent to the first inflatable body and having an outer surface configured to apply an expansion force to the expandable implant and having a shape that tapers downward in a direction towards the first inflatable body to a narrow portion having a second outer diameter that is less than the first outer diameter when the second inflatable body is in an inflated state.
22. The delivery system of embodiment 21, wherein the first inflatable body has a rounded profile when in the inflated state.
23. The delivery system of embodiment 21 or embodiment 22, wherein the first inflatable body includes a shoulder portion configured to impede movement of the expandable implant in a direction towards the first inflatable body.
24. The delivery system of any of embodiments 21-23, wherein the second inflatable body includes a shoulder portion configured to impede movement of the expandable implant in a direction away from the first inflatable body.
25. The delivery system of embodiment 24, wherein the shoulder portion of the second inflatable body comprises a folded portion of the second inflatable body.
26. The delivery system of any of embodiments 21-25, wherein a portion of the first inflatable body overlaps the second inflatable body.
27. The delivery system of any of embodiments 21-26, wherein the first inflatable body includes a first end and a second end, with the first end coupled to the second inflatable body.
28. The delivery system of embodiment 27, wherein the first end of the first inflatable body is coupled to the narrow portion of the second inflatable body.
29. The delivery system of embodiment 28, wherein the first end of the first inflatable body is coupled to the narrow portion of the second inflatable body such that an inflation of the narrow portion causes the first end of the first inflatable body to increase in size.
30. The delivery system of any of embodiments 27-29, wherein the second end of the first inflatable body is coupled to the elongate shaft.
31. The delivery system of any of embodiments 21-30, wherein the second inflatable body includes a first end and a second end each coupled to the elongate shaft.
32. The delivery system of any of embodiments 21-31, wherein the first inflatable body is positioned on the elongate shaft of the delivery apparatus proximal of the second inflatable body.
33. The delivery system of any of embodiments 21-31, wherein the first inflatable body is positioned on the elongate shaft of the delivery apparatus distal of the second inflatable body.
34. The delivery system of any of embodiments 21-33, wherein the elongate shaft includes a distal portion and a proximal portion coupled to a handle, and the second inflatable body and the first inflatable body are each positioned on the distal portion of the elongate shaft.
35. The delivery system of any of embodiments 21-34, further comprising a nose cone positioned distal of the second inflatable body and the first inflatable body.
36. The delivery system of any of embodiments 21-35, further comprising one or more inflation lumens extending along the elongate shaft and configured to inflate one or more of the first inflatable body or the second inflatable body.
37. The delivery system of embodiment 36, wherein the first inflatable body has a first interior chamber, and the one or more inflation lumens are configured to pass fluid into the first interior chamber.
38. The delivery system of embodiment 36 or embodiment 37, wherein the second inflatable body has second interior chamber, and the one or more inflation lumens are configured to pass fluid into the second interior chamber.
39. The delivery system of any of embodiments 21-38, further comprising the expandable implant having a length and configured to be positioned upon the outer surface of the second inflatable body, the expandable implant configured to expand radially outward and have the length shorten in a direction towards the first inflatable body when the outer surface applies the expansion force to the expandable implant.
40. The delivery system of embodiment 39, wherein the expandable implant has a tapered profile.
41. A method comprising:
   inflating a first inflatable body; and
   radially expanding an expandable implant positioned around an outer surface of a second inflatable body by inflating the second inflatable body, the second inflatable body being positioned adjacent to the first inflatable body and having an outer surface having a shape that tapers downward in a direction towards the first inflatable body to a narrow portion, with an outer diameter of the narrow portion being less than an outer diameter of the first inflatable body.
42. The method of embodiment 41, wherein the first inflatable body has a rounded profile when in an inflated state.
43. The method of embodiment 41 or embodiment 42, wherein radially expanding the expandable implant includes shortening a length of the expandable implant in a direction towards the first inflatable body.
44. The method of any of embodiments 41-43, wherein the expandable implant includes a first end and a second end positioned at the narrow portion of the second inflatable body, and the first inflatable body includes a shoulder portion impeding movement of the second end of the expandable implant in a direction towards the first inflatable body.
45. The method of embodiment 44, wherein the shoulder portion of the first inflatable body comprises a folded portion of the first inflatable body.
46. The method of embodiment 44 or embodiment 45, wherein the shoulder portion of the first inflatable body maintains a position of the second end of the expandable implant when the expandable implant is radially expanded.
47. The method of any of embodiments 44-46, wherein the second inflatable body includes a shoulder portion impeding movement of the first end of the expandable implant in a direction away from the first inflatable body.
48. The method of embodiment 47, wherein the shoulder portion of the second inflatable body comprises a folded portion of the second inflatable body.
49. The method of embodiment 47 or embodiment 48, wherein the shoulder portion of the second inflatable body is axially offset from the expandable implant.
50. The method of any of embodiments 41-49, wherein a portion of the first inflatable body overlaps the second inflatable body.
51. The method of any of embodiments 41-50, wherein the first inflatable body includes a first end and a second end, with the first end coupled to the second inflatable body.
52. The method of embodiment 51, wherein the first end of the first inflatable body is coupled to the narrow portion of the second inflatable body.
53. The method of embodiment 52, wherein the first end of the first inflatable body is coupled to the narrow portion of the second inflatable body such that an inflation of the narrow portion causes the first end of the first inflatable body to increase in size.
54. The method of any of embodiments 41-53, wherein the first inflatable body has a portion that is axially offset from the expandable implant.
55. The method of any of embodiments 41-54, wherein the expandable implant has a tapered shape when radially expanded.
56. The method of any of embodiments 41-55, wherein the first inflatable body and the second inflatable body are each coupled to an elongate shaft of a delivery apparatus configured to deliver the expandable implant to a location in a patient's body, and the first inflatable body is positioned proximal of the second inflatable body.
57. The method of any of embodiments 41-55, wherein the first inflatable body and the second inflatable body are each coupled to an elongate shaft of a delivery apparatus configured to deliver the expandable implant to a location in a patient's body, and the first inflatable body is positioned distal of the second inflatable body.
58. The method of any of embodiments 41-57, wherein the first inflatable body has a first interior chamber, and the method further comprises inflating the first interior chamber of the first inflatable body with an inflation lumen.
59. The method of embodiment 58, wherein the second inflatable body has a second interior chamber, and the method further comprises inflating the second interior chamber of the second inflatable body with an inflation lumen.
60. The method of embodiment 59, wherein the second interior chamber is sealed from the first interior chamber.

## Claims

1. A system for expansion of an expandable implant (12), the system comprising:
a first inflatable body (14) having a first outer diameter (28) when in an inflated state; and
a second inflatable body (16) positioned adjacent to the first inflatable body (14) and having an outer surface configured to apply an expansion force to the expandable implant (12) and having a shape that tapers downward in a direction towards the first inflatable body (14) to a narrow portion (32) having a second outer diameter (52) that is less than the first outer diameter (28) when the second inflatable body (16) is in an inflated state,
wherein the first inflatable body (14) has a first interior chamber (38),
wherein the second inflatable body (16) has a second interior chamber (54),
wherein the system further comprises a single inflation lumen for passing fluid into the first interior chamber (38) and the second interior chamber (54).

2. The system of claim 1, wherein the inflation lumen includes a device that allows for selective inflation of the inflatable bodies (14, 16).

3. The system of claim 1 or 2, wherein the first inflatable body (14) and the second inflatable body (16) are comprised of a unitary body.

4. The system of any of claims 1 to 3, wherein the first inflatable body (14) is made from a material that more easily inflates than the second inflatable body (16).

5. The system of any of claims 1 to 4, wherein a portion of the first inflatable body (14) overlaps the second inflatable body (16).

6. The system of any of claims 1 to 5, wherein the first inflatable body (14) has a bulb shape when in an inflated state.

7. The system of any of claims 1 to 6, wherein the second inflatable body (16) has a conical frustum shape when in an inflated state.

8. The system of any of claims 1 to 7, wherein the first inflatable body (14) includes a shoulder portion (30) configured to impede movement of the expandable implant in a direction towards the first inflatable body (14).

9. The system of claim 8, wherein as the first inflatable body (14) is inflated, the shoulder portion (30) forms an outwardly extending and curved surface extending to an apex (34) of the first inflatable body (14).

10. The system of claim 9, wherein the first inflatable body (14) includes a downwardly tapering portion (36) on the opposite side of the apex (34), extending downward to a second end (24) of the first inflatable body (14).

11. The system of claim any of claims 8 to 10, wherein the shoulder portion (30) of the first inflatable body (14) comprises a folded portion of the first inflatable body (14).

12. The system of any of claims 1 to 11, wherein the second inflatable body (16) includes a shoulder portion (50) configured to impede movement of the expandable implant in a direction away from the first inflatable body (14).

13. The system of claim 12, wherein the shoulder portion (50) of the second inflatable body (16) comprises a folded portion of the second inflatable body (16).

14. The system of any of claims 1 to 13, wherein the first inflatable body (14) includes a first end (22) and a second end (24), with the first end (22) coupled to the second inflatable body (16).

15. The system of claim 14, wherein the second end (24) of the first inflatable body (14) is coupled to an elongate shaft (26) of a delivery apparatus configured to deliver the expandable implant to a location in a patient's body.
